Europäisches Patentamt

European Patent Office

Office européen des brevets

(11) Publication number: **0 251 464**
**A2**

(12) **EUROPEAN PATENT APPLICATION**

(21) Application number: **87304364.0**

(22) Date of filing: **18.05.87**

(51) Int. Cl.³: **A 01 N 25/04**
**A 01 N 25/24, A 01 N 53/00**

(30) Priority: **27.05.86 GB 8612826**

(43) Date of publication of application:
**07.01.88 Bulletin 88/1**

(84) Designated Contracting States:
**AT BE DE ES FR GB IT NL SE**

(71) Applicant: **The Boots Company PLC**
**1 Thane Road West**
**Nottingham Nottinghamshire NG2 3AA(GB)**

(72) Inventor: **Keating, David Anthony**
**90 Ullswater Crescent**
**Bramcote Nottingham(GB)**

(74) Representative: **Thacker, Michael Anthony et al,**
**THE BOOTS COMPANY PLC Patents Section R4**
**Pennyfoot Street**
**Nottingham NG2 3AA(GB)**

(54) Composition for the control of insects.

(57) A composition for the control of insects in the form of a substantially anhydrous gel comprises a pyrethrin or pyrethroid active ingredient, a solvent carrier, a carboxyvinyl polymer and a neutralizing agent for the carboxyvinyl polymer. Preferably, the gel evaporates to leave a film of material with insecticidal and insect-repelling properties. The gel has sufficient viscosity that it may be applied to precise locations on the skin.

EP 0 251 464 A2

Croydon Printing Company Ltd.

they do not have the required consistency to allow the area of skin to which the formulations may be applied to be precisely controlled. It is also known to mix insecticides with carriers such as kerosine or other paraffinic materials. However, this produces a greasy film which is undesirable for topical application.

It has now been found that insecticidal ingredients can be formulated with an anhydrous solvent base to provide a composition suitable for topical application to selected areas of the skin.

Accordingly, the invention provides a composition for the control of insects in the form of a substantially anhydrous gel comprising a pyrethrin or pyrethroid active ingredient, a solvent carrier, a carboxyvinyl polymer and a neutralising agent for the carboxyvinyl polymer.

A composition according to the invention may be provided in the form of a cosmetically acceptable gel which is clear and bright. When the gel is provided with a volatile component, on application of the gel to the skin the volatile portion evaporates to leave a non-visible film of material which has advantageous insecticidal and insect repellent properties. The gel is non-greasy, is easy to apply and has sufficient viscosity that it may be applied to precise locations on the skin. A further advantage of the anhydrous composition is that it is stable on storage and has a good shelf-life.

Pyrethrins may be derived from naturally occurring products; pyrethroids may be derived synthetically. Both have effective insect-controlling properties, especially the pyrethroids which have excellent insecticidal and insect-repelling characteristics and

A Composition for the Control of Insects

The invention relates to a composition for the control of insects, and in particular to an anhydrous composition for the control of insects which is suitable for topical application.

Pyrethrins and pyrethroids are known to be very effective insecticidal and insect-repelling agents (see Kirk-Othmer, Encylopedia of Chemical Technology, Third Ed., vol. 13, pages 424-425 and 456-458). It is desired to formulate a composition containing either of these active ingredients for application to selected areas of the skin of a human or animal body to repel and/or kill insects. Advantageously, the composition should be presented in the form of a viscous solution or gel which may be applied to precise locations on the skin. If an aqueous gel system is employed, however, decomposition of the active ingredient may occur over a period of time, leading to undesirable stability properties or a reduction in efficacy. Accordingly, it is desired to present the pyrethrin or pyrethroid ingredient in an anhydrous gel.

Conventional topical cosmetic and therapeutic formulations are usually based on aqueous gels. Such gels are not applicable to the present system. Anhydrous formulations containing these active ingredients have been proposed, for example, it is known to combine insecticides with propellants to be used as spray on formulations. In addition, European Patent Publication No. 45424 discloses a liquid formulation effective against ticks which may comprise a pyrethroid as the active insecticidal ingredient combined with particular solvents adapted to spread the formulation over the skin. However, these formulations are not desirable for topical application on a human as

also a very low toxicity. Preferred active ingredients are permethrin, resmethrin, bioresmethrin, deltamethrin, cyfluthrin, fenfluthrin, decamethrin, tetramethrin and allethrin. The most preferred active ingredient is permethrin. If desired two or more pyrethrins and/or pyrethroids may be employed, or optionally the active ingredient may be combined with known synergists, for example piperonyl butoxide.

Preferably the pyrethrin or pyrethroid may be used in a composition according to the invention up to an extent of 2%. Conveniently the composition comprises 0.1-1% by weight pyrethrin or pyrethroid, more preferably 0.4-0.6% by weight.

The anhydrous gelling system comprises a gelling system which does not require the presence of water to be effective. The anhydrous gelling system comprises a carboxyvinyl polymer in combination with an anhydrous neutralising agent. On addition of the neutralising agent to a solution of carboxyvinyl polymer, the reaction between the components causes the viscosity of the solution to increase and a gel to be formed. The amount of carboxyvinyl polymer present is sufficient to thicken the composition to produce a suitable gel, i.e. one that is not too fluid, but also is not so thick that it is difficult to apply topically or after evaporation of any volatile component leaves a thick film on the skin. If the film left on the skin is too thick it may be unsightly and may hinder the action of the active ingredient. The carboxyvinyl polymers suitable for use in a composition according to the invention are preferably acrylic acid polymers cross-linked with allylsucrose. One source of such polymers is the range of polymers available from B.F. Goodrich Chemical Company under the Trade Name Carbopol. The most preferred polymer for use in an invention according to the invention is the polymer available under the Trade Name Carbopol 940. Depending on the mode of application of the anhydrous gel to a human or animal, the carboxyvinyl polymer may be employed up to an extent of 3% by weight or greater. A higher content of carboxyvinyl polymer in the composition is capable of producing a more viscous gel. Preferably the composition contains 0.1-1% by weight of carboxyvinyl polymer. When the composition is presented in the form of a semi-solid gel, for example from a tube, it is preferred to add about 0.5% by weight of the composition of carboxyvinyl polymer, however when it is desired to present the composition in a roll-on container, suitably the composition is less viscous and advantageously about 0.2% by weight carboxyvinyl polymer is added.

The neutralising agent employed in combination with the carboxyvinyl polymer causes the carboxyvinyl polymer to thicken and form a gel having an adequate consistency. A gel is formed even if there is only partial neutralisation of the carboxyvinyl polymer; such a gel will be less viscous than when the neutralisation of the polymer is complete. Suitably the carboxyvinyl polymer and the neutralising agent are used in a ratio of 0.5:1-2:1 parts by weight, especially 0.5:1-1:1 parts by weight. Any suitable compound which combines with the carboxyvinyl polymer to increase the viscosity of the composition may be employed. In order to be suitable for use in an anhydrous composition according to the invention, the neutralising agent is also anhydrous. Suitable neutralising agents are found in the amine class. When the neutralising agent is added to the carboxyvinyl polymer, the salt formed should be soluble in the carrier to form a cosmetically acceptable gel. Especially advantageous results are obtained by the use of N,N,N',N'-tetrakis(2-hydroxypropyl)ethylenediamine as the neutralising agent. When this neutralising agent is used the components form a clear solution; the components do not form a precipitate which would cause the production of an unsatisfactory gel and thus prevent the production of a clear, bright gel.

The solvent carrier comprises any suitable anhydrous solvent for the above-mentioned ingredients. As the composition is primarily desired for application to the skin of a human, the solvent should not be irritant to the skin. The solvent is preferably polar and especially has hydroxy groups which enhance the thickening effect of the carboxyvinyl polymer. The carrier may comprise an alcohol component. The alcohol component should not, however, be present in such a large amount as to introduce the disadvantages of flammability or irritancy to the skin or require the addition of large amounts of carboxyvinyl polymer in order to reduce the fluidity of the composition. Suitable carriers include a selection of one or more of the following: alcohols, glycols and polyglycols. Examples of suitable alcohols include ethanol, isopropyl alcohol, n-propanol, diacetone alcohol, butanol, octanol, 2-octyldodecanol, oleyl alcohol, amyl alcohol, cetyl stearyl alcohol and benzyl alcohol. Examples of suitable glycols include ethylene glycol, propylene glycol and 1,3 butylene glycol. Examples of suitable polyglycols include polyethylene glycol, polypropylene glycol, ethyl hexane diol and glycerin. The carrier may comprise between 55 and 99% by weight of the composition, suitably between 75 and 99% and preferably between 85 and 95% by weight.

Preferred anhydrous compositions have a significant amount of glycerin in the carrier. In an especially preferred composition according to the invention the carrier comprises a combination of ethanol, glycerin and propylene glycol. Glycerin has advantageous emollient properties. The ethanol and propylene glycol are added in sufficient proportions that they evaporate quickly on the skin to leave an effective amount of insect-controlling film on the skin. Preferably the ethanol and glycerin are each employed in the range of 25 to 60% by weight of the composition and the propylene glycol is employed in the range of 5-20% by weight of the composition. Most suitably similar quantities of glycerin and ethanol are employed. The ratio of glycerin to propylene glycol is preferably in the range 3:1-5:1 parts by weight.

As it may not be possible to obtain each of the components in a composition according to the invention in a totally anhydrous state, it is to be understood that the composition may contain a very small amount of water.

Other known insecticides and insect repellents may be employed in addition to the pyrethrin or pyrethroid as the active ingredient in a composition according to the invention, for example diethyl toluamide and/or one or more phthalates, in particular methyl or butyl phthalate, which are commonly known insect repellents. Suitably the diethyl toluamide and/or phthalate ester may each be used up to an extent of 20% by weight, advantageously 5-15% by weight of the composition.

Further additives as desired may be added to an insecticidal composition according to the invention. Conveniently a perfume ingredient, a sunscreen additive and/or other additives to produce a suitable cosmetic effect may be added. Such additives may include volatile silicones and oleyl alcohol which provide a good "skin feel" and, for example, 2-ethoxyethyl p-methoxycinnamate as a sunscreen agent.

A composition according to the invention may be formed by combining the pyrethrin or pyrethroid with a carboxyvinyl polymer, a neutralising agent for the carboxyvinyl polymer and a solvent carrier and stirring until a gel is formed.

A preferred anhydrous composition according to the invention may be prepared by the following steps.

In the first step the carboxyvinyl polymer is dissolved in diethyl toluamide. Under vacuum a portion of the solvent carrier is added to the diethyl toluamide and mixed thoroughly. The product is allowed to stand until a clear solution is obtained. In the second step the active insecticidal ingredient, optionally with other excipients is dissolved in a further portion of the solvent carrier. In the third step the neutralising agent for the carboxyvinyl polymer is dissolved in the remainder of the solvent carrier. Finally the products obtained from steps 1, 2 and 3 are combined and stirred continuously under vacuum whilst the composition thickens and all entrained air is removed. The resulting product is discharged into a sealed container for example a tube or bottle. In an alternative process, in the first step the carboxyvinyl polymer is dissolved in a portion of the solvent carrier and allowed to stand until a clear solution is formed and in the second step the diethyl toluamide together with the active insecticidal ingredient is dissolved in a further portion of the solvent carrier. In the third step the neutralising agent for the carboxyvinyl polymer is dissolved in the remainder of the solvent carrier. Finally the products obtained from steps 1, 2 and 3 are combined and stirred continuously under vacuum whilst the composition thickens and all entrained air is removed.

The gels of the present invention may vary in consistency between a semi-solid material and a viscous liquid. They may be presented in any suitable container or tube or may be provided in a special dispenser, for example a roll-on dispenser. Preferably the viscosity of the gel is in the range 100-2000 cp, especially 300-1000 cp.

The compositions are primarily intended to be used to control mosquitoes, midges, ticks and lice.

The invention is illustrated by the following non-limitative Examples.

In the Examples the carboxyvinyl polymer is a polymer of acrylic acid cross-linked with allylsucrose and containing 56 to 68% of carboxylic acid groups supplied by B.F. Goodrich, Middlesex, England under the trade name Carbopol 940, the pyrethroid is supplied by Mitchell Cotts, W. Yorkshire, England under the trade name Permethrin Technical; and N,N,N',N'-tetrakis-(2-hydroxypropyl)ethylene diamine is supplied by BASF Wyandotte Corporation, Michigan, USA under the trade name Quadrol or under the trade name Pluriol Q.

Example 1

The example anhydrous composition for the control of insects comprised the following components:-

|  | parts by weight |
|---|---|
| Carboxyvinyl polymer (Carbopol 940) | 0.5 |
| Diethyl Toluamide BP | 5.0 |
| Pyrethroid (Permethrin Technical) | 1.0 |
| N,N,N',N'-tetrakis(2-hydroxy-propyl)ethylene diamine (Pluriol Q) | 0.75 |
| Propylene Glycol BP | 10.0 |
| Glycerin BP | 42.25 |
| Isopropyl Alcohol BP | 40.0 |
| Perfume Component | 0.2 |

The composition was made according to the following steps.

Step 1: The carboxyvinyl polymer was dissolved in diethyl toluamide. Under vacuum the propylene glycol and glycerin were added and mixed thoroughly with the solution until all the entrained air was released. The product was allowed to stand until a clear solution was obtained.

Step 2: The pyrethroid and the perfume component were stirred into and dissolved in half of the isopropyl alcohol BP and the resulting solution added to the glycerin solution obtained in step 1 under vacuum.

Step 3: The N,N,N',N'-tetrakis(2-hydroxypropyl)-ethylene diamine was dissolved in the remainder of the isopropyl alcohol and added to the products produced in steps 1 and 2 under vacuum.

Step 4: The components were stirred continuously whilst the composition thickened and all entrained air was removed. The product was then discharged into sealed tube.

Example 2

The anhydrous composition was formulated in a similar manner to that described in Example 1 using the following components:-

|  | parts by weight |
|---|---|
| Carboxyvinyl polymer (Carbopol 940) | 0.5 |
| Diethyl Toluamide BP | 5.0 |
| Pyrethroid (Permethrin Technical) | 1.0 |
| N,N,N',N'-tetrakis(2-hydroxy-propyl)ethylene diamine (Pluriol Q) | 0.75 |
| Propylene Glycol BP | 10.0 |
| Glycerin BP | 42.25 |
| Denatured Ethanol B Grade 100% | 40.0 |
| Perfume Component | 0.2 |

Example 3

The anhydrous composition having a viscosity of 700 cp was formulated in a similar manner to that described in Example 1 using the following components:-

|  | parts by weight |
|---|---|
| Carboxyvinyl polymer (Carbopol 940) | 0.5 |
| Diethyl Toluamide BP | 5.0 |
| Pyrethroid (Permethrin Technical) | 0.5 |
| N,N,N',N'-tetrakis(2-hydroxy-propyl)ethylene diamine (Quadrol) | 0.75 |
| Propylene Glycol BP | 10.0 |
| Glycerin BP | 43.05 |
| Denatured Ethanol B Grade 100% | 40.0 |
| Perfume Component | 0.2 |

## Example 4

The anhydrous composition was formulated in a similar manner to that described in Example 1 using the following components:-

|  | parts by weight |
|---|---|
| Carboxyvinyl polymer (Carbopol 940) | 0.5 |
| Diethyl Toluamide BP | 5.0 |
| Pyrethroid (Permethrin Technical) | 0.5 |
| N,N,N',N'-tetrakis(2-hydroxy-propyl)ethylene diamine (Quadrol) | 0.75 |
| Propylene Glycol BP | 10.0 |
| Glycerin BP | 43.05 |
| Isopropyl Alcohol BP | 40.0 |
| Perfume Component | 0.2 |

Example 5

The anhydrous composition was formulated in a similar manner to that described in Example 1 using the following components:-

|                                                    | parts by weight |
| -------------------------------------------------- | --------------- |
| Carboxyvinyl polymer (Carbopol 940)                | 0.5             |
| Diethyl Toluamide BP                               | 5.0             |
| Pyrethroid (Permethrin Technical)                  | 0.5             |
| N,N,N',N'-tetrakis(2-hydroxy-propyl)ethylene diamine (Quadrol) | 0.75 |
| Polyethylene Glycol 400                            | 10.0            |
| Glycerin BP                                        | 43.05           |
| Denatured Ethanol B Grade 100%                     | 40.0            |

Example 6

The anhydrous composition having a viscosity of 310 cp was formulated in a similar manner to that described in Example 1 using the following components:-

|                                           | parts by weight |
| ----------------------------------------- | --------------- |
| Carboxyvinyl polymer<br>(Carbopol 940)    | 0.2             |
| Diethyl Toluamide BP                      | 5.0             |
| Propylene Glycol BP                       | 10.0            |
| Glycerin BP                               | 41.8            |
| Volatile Silicone Fluid Y-7158            | 2.0             |
| Denatured Ethanol B Grade 100%            | 40.0            |
| Perfume                                   | 0.2             |
| Pyrethroid (Permethrin Technical)         | 0.5             |
| N,N,N',N'-tetrakis(2-hydroxy-<br>propyl)ethylene diamine<br>(Quadrol) | 0.3 |

Example 7

The effectiveness of the anhydrous compositions according to the invention were determined using mosquitoes as follows:-

The compositions of Examples 1 and 3 were applied lightly but evenly to test skin areas on a human forearm on a piece of cotton wool. Excess was quickly wiped away with one or more clean pieces of cotton wool such that the arm surface showed no visible trace of product. Four trials were carried out at 28°C for each of examples 1 and 3. The repellency time was calculated from when the test skin area was exposed to a mosquito-infested environment until the first mosquito bite was observed. Table 1 shows the repellency time in minutes for each of the trials. The trials were discontinued after 240 minutes, so insect repellent times of greater than 240 minutes show that the anhydrous composition was still effective at the time the experiment was discontinued.

## Table 1

|  | Time (mins) | |
| --- | --- | --- |
|  | Ex. 1 | Ex. 3 |
| Trial 1 | 240 | >240 |
| Trial 2 | >240 | >240 |
| Trial 3 | >240 | 190 |
| Trial 4 | 180 | 210 |

## Claims

1. A composition for the control of insects in the form of a substantially anhydrous gel comprising a pyrethrin or pyrethroid active ingredient, a solvent carrier, a carboxyvinyl polymer and a neutralising agent for the carboxyvinyl polymer.

2. A composition according to claim 1 wherein the neutralising agent comprises N,N,N',N'-tetrakis(2-hydroxypropyl)ethylene diamine.

3. A composition according to either one of claims 1 and 2 wherein the carboxyvinyl polymer comprises 0.1 to 1% by weight of the composition.

4. A composition according to any one of claims 1 to 3 wherein the solvent carrier comprises one or more of an alcohol, a glycol or a polyglycol.

5. A composition according to any one of claims 1 to 4 wherein the carrier comprises ethanol, glycerin and propylene glycol.

6. A composition according to any one of the preceding claims wherein the solvent carrier comprises between 75 and 99% by weight of the composition.

7. A composition according to any one of the preceding claims wherein the active ingredient comprises permethrin.

8. A composition according to any one of the preceding claims wherein the pyrethrin or pyrethroid comprises 0.1-1% by weight of the composition.

9. A composition according to any one of the preceding claims comprising a further insecticide or insect repellent active ingredient.

10. A process to prepare a composition as claimed in any one of the preceding claims in which the pyrethrin or pyrethroid is combined with a carboxyvinyl polymer, a neutralising agent for the carboxyvinyl polymer and a solvent carrier and stirred until a gel is formed.

## Claims for Spain

1. A process to prepare a composition for the control of insects in the form of a substantially anhydrous gel comprising a pyrethrin or pyrethroid active ingredient, a solvent carrier, a carboxyvinyl polymer and a neutralizing agent for the carboxyvinyl polymer in which the pyrethrin or pyrethroid is combined with the carboxyvinyl polymer, the neutralizing agent for the carboxyvinyl polymer and the solvent carrier and stirred until a gel is formed.

2. A process according to claim 1 wherein the neutralizing agent comprises N,N,N',N'-tetrakis(2-hydroxypropyl)ethylene diamine.

3. A process according to either one of claims 1 and 2 wherein the carboxyvinyl polymer comprises 0.1 to 1% by weight of the composition.

4. A process according to any one of claims 1 to 3 wherein the solvent carrier comprises one or more of an alcohol, a glycol or a polyglycol.

5. A process according to any one of claims 1 to 4 wherein the carrier comprises ethanol, glycerin and propylene glycol.

6. A process according to any one of the preceding claims wherein the solvent carrier comprises between 75 and 99% by weight of the composition.

7. A process according to any one of the preceding claims wherein the active ingredient comprises permethrin.

8. A process according to any one of the preceding claims wherein the pyrethrin or pyrethroid comprises 0.1-1% by weight of the composition.

9. A process according to any one of the preceding claims comprising a further insecticide or insect repellent active ingredient.

10. A process to prepare a composition as claimed in any one of the preceding claims comprising the steps of

a)   combining the carboxyvinyl polymer with a portion of the solvent carrier;

b)   combining the pyrethrin or pyrethroid active ingredient with a further portion of the solvent carrier;

c)   dissolving the neutralizing agent in a further portion of the solvent carrier;   and

d)   combining the products from steps (a), (b) and (c) and stirring until a gel is formed.